# FASCICULE DE BREVET EUROPEEN

(11) **EP 1 357 890 B1**
(45) Date de publication et mention de la délivrance du brevet: **28.12.2005**
(21) Numéro de dépôt: 02700361.5
(22) Date de dépôt: 22.01.2002
(51) Int. Cl.: A61K 7/135, A61K 7/09, C01B 15/00

(54) **COMPOSITION OXYDANTE POUR LE TRAITEMENT DES MATIERES KERATINIQUES COMPRENANT UN POLY(VINYLLACTAME) CATIONIQUE**
OXYDIERENDE ZUSAMMENSETZUNG ZUR BEHANDLUNG VON KERATINFASERN, DIE EIN KATIONISCHES POLYVINYLLACTAM ENTHALTEN
OXIDISING COMPOSITION FOR THE TREATMENT OF KERATIN COMPRISING A CATIONIC POLY(VINYLLACTAM)

(30) Priorité: 26.01.2001 FR 0101113
(43) Date de publication de la demande: 05.11.2003
(73) Titulaire: L'OREAL, 75008 Paris (FR)
(72) Inventeur: LEGRAND, Frédéric, F-92400 Courbevoie (FR); DE LA METTRIE, Roland, F-78110 Le Vésinet (FR)
(74) Mandataire: Wattremez, Catherine
(86) Numéro de dépôt international: PCT/FR2002/000252
(87) Numéro de publication internationale: WO 2002/058660

(56) Documents cités:
- EP-A- 0 275 153
- EP-A- 0 351 772
- WO-A-00/68282
- WO-A-93/14024
- GB-A- 747 806
- US-A- 4 614 646
- US-A- 5 143 518
- US-A- 5 190 749

## Description

La présente invention a trait à une composition oxydante gélifiée, destinée au traitement des matières kératiniques comprenant un poly(vinyllactame) cationique ainsi que ses utilisations pour la teinture, pour la déformation permanente ou pour la décoloration des fibres kératiniques humaines et en particulier des cheveux.

Il est connu de décolorer les fibres kératiniques et en particulier les cheveux humains, avec des compositions de décoloration contenant un ou plusieurs agents oxydants. Parmi les agents oxydants classiquement utilisés, on peut citer le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Lesdites compositions de décoloration se présentent principalement sous forme de produits anhydres (poudres ou crèmes) contenant des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.
Les compositions de décoloration peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.
Les compositions de décoloration se présentent également sous forme de compositions aqueuses épaissies de peroxyde d'hydrogène, prêtes à l'emploi.

Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

Il est connu par ailleurs de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants. On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.
L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène ou des composés susceptibles de produire le peroxyde d'hydrogène par hydrolyse tels que le peroxyde d'urée, les persels comme les perborates et persulfates. Le peroxyde d'hydrogène est particulièrement préféré.

Pour localiser le produit de décoloration ou de teinture à l'application sur les cheveux afin qu'il ne coule pas sur le visage ou en dehors des zones que l'on se propose de décolorer, on a jusqu'ici eu recours à l'emploi d'épaississants traditionnels tels que l'acide polyacrylique réticulé, les hydroxyéthylcelluloses, certains polyuréthanes, les cires, et en outre, dans le cas des compositions aqueuses de décoloration, à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.
Cependant, la demanderesse a constaté que les systèmes épaississants mentionnés ci-dessus ne permettaient pas d'obtenir des décolorations suffisamment puissantes et homogènes et laissaient les cheveux rêches.
Par ailleurs, elle a également constaté que les compositions de décoloration prêtes à l'emploi contenant le ou les agents oxydants, et en outre les systèmes épaississants de l'art antérieur ne permettaient pas une application suffisamment précise sans coulures ni chutes de viscosité dans le temps.

On sait également que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.
Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique ou l'acide thioglycolique, leurs sels ainsi que leurs esters, notamment le thioglycolate de glycérol.
Concernant les compositions oxydantes nécessaires à la mise en oeuvre de l'étape de fixation, on fait le plus souvent appel, dans la pratique, à des compositions à base d'eau oxygénée.

Par ailleurs, on recherche depuis de nombreuses années des formulations cosmétiques sous forme de gels. Ce type de présentation est très appréciée par le consommateur pour des raisons esthétiques et pour des raisons de facilité et de confort d'utilisation.
La forme gel correspond le plus souvent à une préoccupation pratique pour le formulàteur : faciliter la prise du produit hors de son conditionnement sans perte significative, limiter la diffusion du produit à la zone locale de traitement et pouvoir l'utiliser dans des quantités suffisantes pour obtenir l'effet cosmétique recherché. Cet objectif est important pour les formulations oxydantes utilisées en teinture capillaire, pour les permanentes ou pour la décoloration des cheveux. Celles-ci doivent bien s'étaler et se répartir de façon régulière le long des fibres kératiniques et ne pas couler sur le front, la nuque, le visage ou dans les yeux.
Il est généralement difficile d'obtenir des gels à base de peroxyde tel que le peroxyde d'hydrogène stables à la conservation en utilisant des épaississants et/ou des gélifiants hydrosolubles classiques, par exemple ceux du type polymère acrylique réticulé comme ceux vendus sous le nom CARBOPOL® par la société GOODRICH. Les peroxydes sont utilisés en cosmétique sous forme de solutions aqueuses acides pour des raisons de stabilité. lls conduisent le plus souvent en présence des gélifiants classiques à des variations sensibles de la viscosité du gel durant le stockage.

On connaît dans le brevet US 4.804.705 des gels à base de peroxyde d'hydrogène contenant un gélifiant formé par réaction d'une hydroxyéthylcellulose quaternisée du type CELQUAT® (produit vendu par NATIONAL STARCH), d'une solution aqueuse à 15% en poids d'un polymère poly(acide 2-acrylamido 2-méthylpropane sulfonique) non-réticulé du type COSMEDIA® HSP-1180 (produit vendu par HENKEL) et d'un polystyrène sulfonate de sodium du type FLEXAN® 3 (produit vendu par NATIONAL STARCH), utilisés dans des concentrations particulières.

La demanderesse a découvert de manière surprenante qu'il est possible d'obtenir des compositions de décoloration prêtes à l'emploi qui ne coulent pas et restent donc bien localisées au point d'application, et qui permettent aussi d'obtenir des décolorations puissantes et homogènes tout en laissant les cheveux moins rêches si on introduit dans la composition une quantité efficace d'un poly(vinyllactame) cationique.

La demanderesse a également découvert de manière surprenante une nouvelle famille d'agents épaississants et/ou gélifiants permettant d'obtenir des gels à base d'oxydants et de préférence de peroxyde d'hydrogène ou de composé oxydant susceptible de libérer du peroxyde d'hydrogène, stables au stockage. Il s'agit de polymères poly(vinyllactame) cationiques. Ces nouveaux agents gélifiants n'affectent pas les propriétés oxydantes du peroxyde d'hydrogène ou d'un composé susceptible de produire le peroxyde d'hydrogène par hydrolyse présent dans le gel ainsi formé.

La présente invention a donc pour objet une composition cosmétique et/ou dermatologique destinée au traitement des matières kératiniques comprenant dans un support approprié pour les matières kératiniques :
(i) au moins un agent oxydant et,
(ii) au moins un poly(vinyllactame) cationique.

L'invention concerne également l'utilisation de ces poly(vinyllactame) cationiques comme agent épaississant et/ou gélifiant dans des compositions cosmétiques et/ou dermatologiques comprenant au moins un oxydant.

Selon l'invention l'agent oxydant est de préférence choisi dans le groupe constitué par le peroxyde d'hydrogène et les composés susceptibles de produire par hydrolyse du peroxyde d'hydrogène, ou leurs mélanges.

Mais d'autres caractéristiques, aspects, objets et avantages de l'invention apparaîtront encore plus clairement à la lecture de la description et des exemples qui suivent.

Sans vouloir être lié par une quelconque théorie, il semblerait que les avantages apportés par les poly(vinyllactame) cationiques selon la présente invention et tels que définis ci-après soient en relation avec un comportement de polymères épaississants de type associatif.
Les polymères associatifs sont des polymères dont les molécules sont capables, dans le milieu de formulation, de s'associer entre elles ou avec des molécules d'autres composés.
Leur structure chimique comprend généralement au moins une zone hydrophile et au moins une zone hydrophobe, la ou les zones hydrophobes comprenant au moins une chaîne grasse.

### Polymères polyvinyllactames cationiques selon l'invention

Les polymères poly(vinyllactame) cationiques selon l'invention comprennent :
- a) au moins un monomère de type vinyl lactame ou alkylvinyllactame;
- b) au moins un monomère de structures (I) ou (II) suivantes : 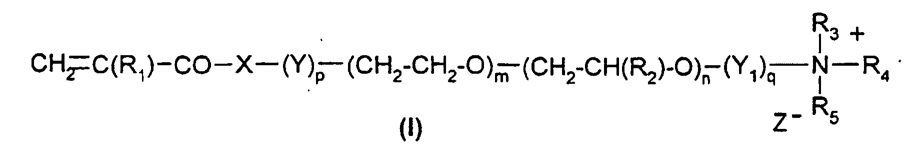 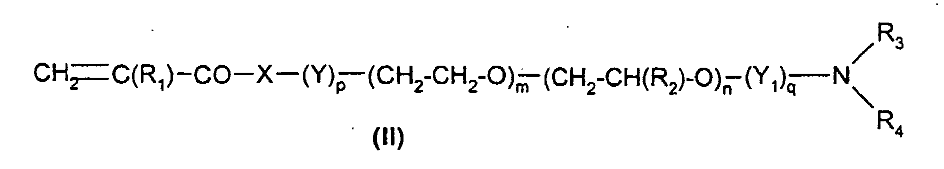
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (III) :

   ―(Y₂)ᵣ―(CH₂-CH(R₇)-O)ₓ―R₈ (III)
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence le contre ion Z⁻ des monomères de formule (I) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.
Plus préférentiellement, le monomère b) est un monomère de formule (I) pour laquelle, encore plus préférentiellement, m et n sont égaux à zéro.

Le monomère vinyl lactame ou alkylvinyllactame est de préférence un composé de structure (IV) : 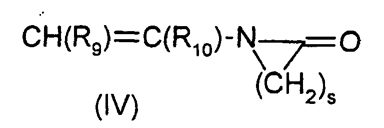 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (IV) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (IV),
b)-un monomère de formule (I) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (II) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,1 à 55% de monomère (c) et 0,25 à 50% de monomère (b). De tels polymères sont décrits dans la demande de brevet WO-00/68282.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone /diméthylaminopropylméthacrylamide/ tosylate de cocoyldiméthylméthacrylamidopropylammonium, les terpolymères vinylpyrrolidone / diméthylaminopropylméthacrylamide / tosylate ou chlorure de lauryldiméthyiméthacrylamidopropylammonium.

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

Les polymères poly(vinyllactame) cationiques conformes à l'invention sont présents dans les compositions dans des concentrations allant de 0,01 à 30% en poids, plus préférentiellement de 0,1 à 10%, et plus particulièrement encore de 0,5 à 2% en poids.

### Agent oxydant

L'agent oxydant de la composition selon l'invention est choisi de préférence dans le groupe formé par l'eau oxygénée, le peroxyde d'urée, les persels tels que les perborates ou les persulfates ou leurs mélanges.

De préférence, l'agent oxydant est le peroxyde d'hydrogène, et plus préférentiellement encore, l'agent oxydant est de l'eau oxygénée.

La concentration en peroxyde d'hydrogène peut varier de 0,5 à 40 volumes, de préférence de 2 à 30 volumes et celle en composé susceptible de former du peroxyde d'hydrogène par hydrolyse de 0,1 à 25 % en poids par rapport au poids total de la composition oxydante.

Les compositions oxydantes selon l'invention peuvent être anhydres ou aqueuses.

Les compositions oxydantes selon l'invention sont de préférence aqueuses et le pH de l'ensemble d'une composition oxydante aqueuse varie de préférence de 1 à 13, et encore plus préférentiellement de 2 à 12.

La composition oxydante peut également se présenter, en particulier dans le cas de la décoloration, sous forme de deux parties à mélanger au moment de l'emploi, l'une de ces deux parties contenant des agents alcalins et se présentant sous forme solide ou liquide. Pour le peroxyde d'hydrogène, le pH est de préférence inférieur à 7 avant mélange.

Le pH des compositions aqueuses oxydantes selon l'invention peut être obtenu et/ou ajusté classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

La composition oxydante peut contenir des additifs connus pour leur utilisation dans les compositions oxydantes pour la teinture des cheveux par oxydation, pour la déformation permanente ou la décoloration des cheveux tels que des agents alcalinisants ou acidifiants, des agents conservateurs, ou des agents séquestrants.

De préférence, lorsque l'agent oxydant est de l'eau oxygénée, la composition oxydante selon l'invention contient au moins un agent stabilisant de l'eau oxygénée. Dans les compositions associant l'eau oxygénée et les polymères amphiphiles de la présente invention, des résultats particulièrement avantageux ont été obtenus en utilisant au moins un agent stabilisant choisi parmi les pyrophosphates des métaux alcalins ou alcalino-terreux, les stannates des métaux alcalins ou alcalino-terreux, la phénacétine ou les sels d'acides et d'oxyquinoléine, comme le sulfate d'oxyquinoléine. De manière plus avantageuse encore, on utilise au moins un stannate en association ou non à au moins un pyrophosphate.
Dans les compositions oxydantes selon l'invention, la concentration en agents stabilisants de l'eau oxygénée peut varier de 0,0001% à 5% en poids et de préférence de 0,01 à 2% en poids par rapport au poids total des compositions oxydantes.
Dans les compositions oxydantes selon l'invention à l'eau oxygénée, le rapport des concentrations du peroxyde d'hydrogène aux agents stabilisants peut varier de 0,05 à 1000 et de préférence de 0,1 à 500 et plus préférentiellement encore de 1 à 200. De même, le rapport des concentrations du ou des polymères poly(vinyllactame) cationiques selon l'invention aux agents stabilisants peut varier de 0,05 à 1000 et de préférence de 0,1 à 500 et plus préférentiellement encore de 1 à 200.
De préférence, selon l'invention, le rapport des concentrations du ou des poly(vinyllactame) cationiques selon l'invention aux agents oxydants est compris entre 0,001 et 10, les quantités desdits polymères et oxydants étant exprimées en matières actives (peroxyde d'hydrogène pour l'eau oxygénée). Plus préférentiellement encore, ce rapport est compris entre 0,01 et 5 et plus particulièrement encore entre 0,02 et 1.

Plus particulièrement, les compositions selon l'invention peuvent contenir en outre au moins un polymère amphotère ou un polymère cationique différent des poly(vinyllactame) cationiques selon la présente invention.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 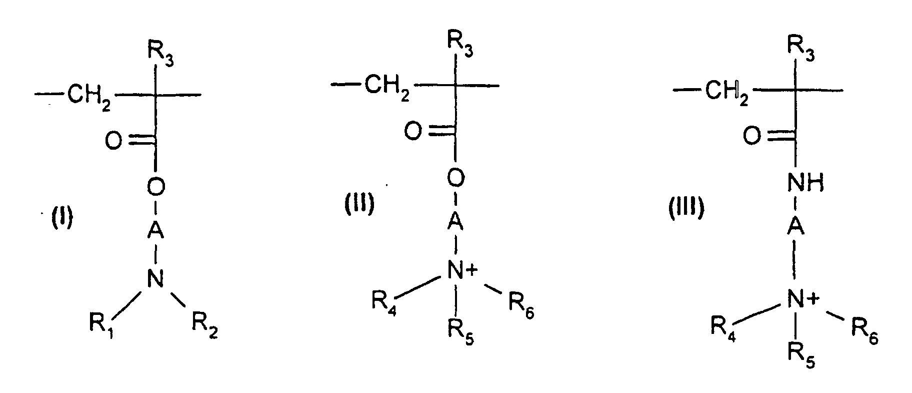 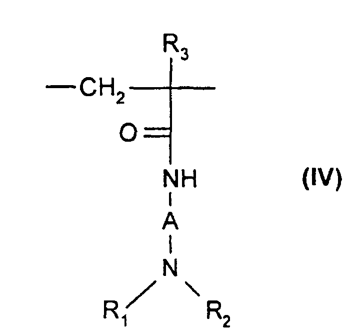 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone :
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC® par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100® par la société CIBA GEIGY,
   - le copolymère d'acrylamide et de méthosulfate de méthacryloyloxyéthyltriméthylammonium vendu sous la dénomination RETEN® par la société HERCULES,
   - les copolymères vinylpyrrolidone / acrylate ou méthacrylate de dialkylaminoalkyle quaternisés ou non, tels que les produits vendus sous la dénomination "GAFQUAT®" par la société ISP comme par exemple "GAFQUAT 734" ou "GAFQUAT 755" ou bien les produits dénommés "COPOLYMER® 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713® par la société ISP,
   - les copolymère vinylpyrrolidone / méthacrylamidopropyl dimethylamine commercialisés notamment sous la dénomination STYLEZE CC 10® par ISP,
   - et les copolymères vinylpyrrolidone / méthacrylamide de diméthylaminopropyle quatemisés tel que le produit vendu sous la dénomination "GAFQUAT® HS 100" par la société ISP .
(2) Les dérivés d'éthers de cellulose comportant des groupements ammonium quaternaire décrits dans le brevet français 1 492 597, et en particulier les polymères commercialisés sous les dénominations "JR®" (JR 400, JR 125, JR 30M) ou "LR®" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat® L 200" et "Celquat® H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR® C13 S, JAGUAR® C 15, JAGUAR® C 17 ou JAGUAR® C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine® F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett® 57" par la société Hercules Inc. ou bien sous la dénomination de "PD 170®" ou "Delsette® 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 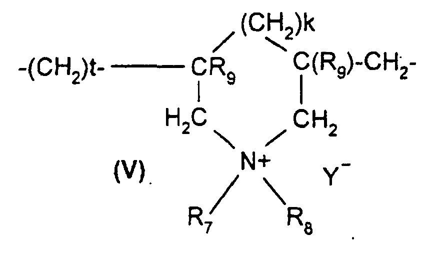 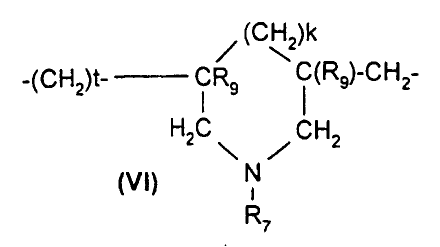 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat® 100" par la société Calgon (et ses homologues de faible masse moléculaire moyenne en poids) et les copolymères de chlorure de diallyldiméthylammonium et d'acrylamide commercialisés sous la dénomination "MERQUAT® 550".
(10) Le polymère de diammonium quaternaire contenant des motifs récurrents répondant à la formule : 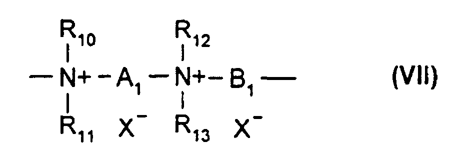 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 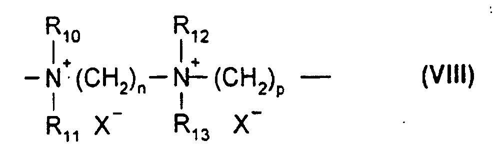 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polyammoniums quaternaires constitués de motifs récurrents de formule (IX) : 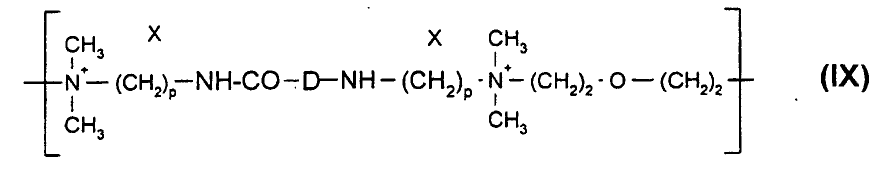 dans laquelle p désigne un nombre entier variant de 1 à 6 environ, D peut être nul ou peut représenter un groupement -(CH₂)ᵣ -CO- dans lequel r désigne un nombre égal à 4
   ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol®A 15", "Mirapol® AD1", "Mirapol® AZ1" et "Mirapol® 175" vendus par la société Miranol.
(12) Les polymères quaternaires de vinylpyrrolidone et de vinylimidazole tels que par exemple les produits commercialisés sous les dénominations Luviquat® FC 905, FC 550 et FC 370 par la société B.A.S.F.
(13) Les polyamines comme le Polyquart H® vendu par HENKEL, référencé sous le nom de "POLYETHYLENEGLYCOL (15) TALLOW POLYAMINE" dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 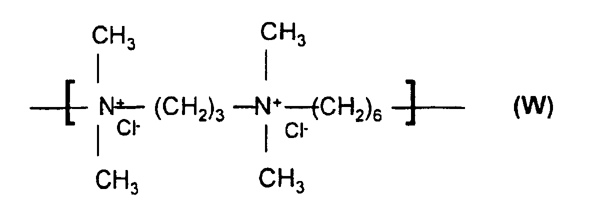 et notamment ceux dont le poids moléculaire, déterminé par chromatographie par perméation de gel, est compris entre 9500 et 9900; 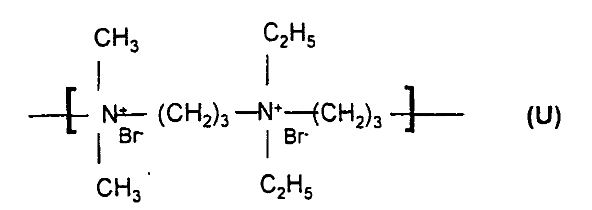 et notamment ceux dont le poids moléculaire, déterminé par chromatographie par perméation de gel, est d'environ 1200.

La concentration en polymère cationique différent des poly(vinyllactame) cationiques selon l'invention dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART® KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT® 280, MERQUAT® 295 et MERQUAT® PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER® ou LOVOCRYL® 47 par la société NATIONAL STARCH.
(3) Les polyaminoamides réticulés et alcoylés partiellement ou totalement dérivant de polyaminoamides de formule générale :

   ⁅CO―R₁₉-CO―Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 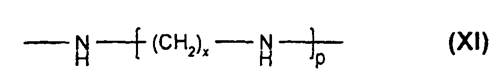 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 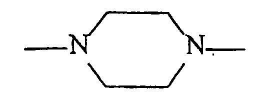
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent, réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium
   ou de potassium.
(4) Les polymères comportant des motifs zwittérioniques de formule : 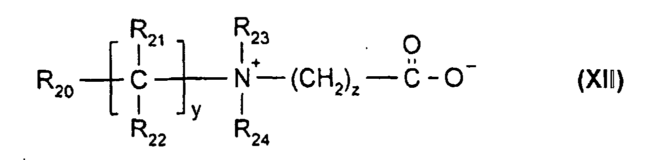 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10. Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301® par la société SANDOZ.
(5) les polymères dérivés du chitosane comportant des motifs monomères répondant aux formules (XIII), (XIV), (XV) suivantes : 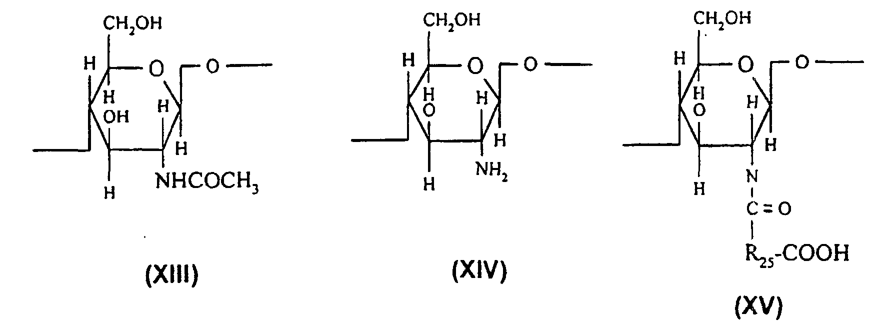 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 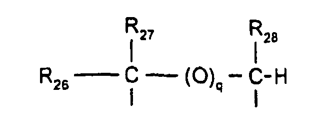 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN®" par la société JAN DEKKER.
(7) Les polymères répondant à la formule générale (XI) tels que ceux décrits par exemple dans le brevet français 1 400 366 : 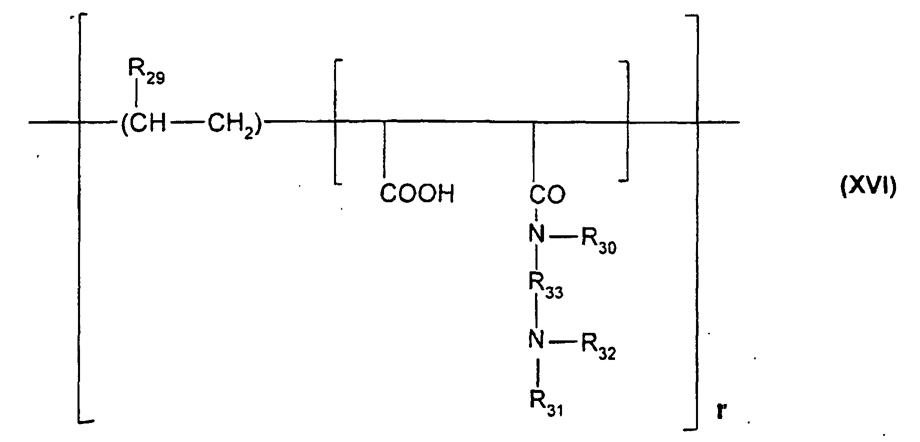 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 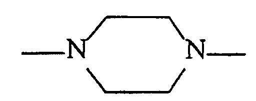 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)
   où D désigne un radical 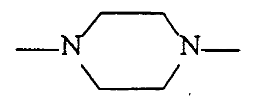 et X désigne le symbole E ou E' et au moins une fois E'; E ayant la signifcation indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs. Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates les alkylsulfonates, alkylphosphates, alkylamidesulfonates, alkylarylsulfonates, α-oléfine-sulfonates, paraffine-sulfonates ; les alkyl(C₆-C₂₄) suffosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

### Agents épaississants additionnels

Les compositions selon l'invention peuvent également contenir d'autres agents d'ajustement de la rhéologie tels que les épaississants cellulosiques (hydroxyéthycellulose, hydroxypropylcellulose, carboxyméthylcellulose..), la gomme de guar et ses dérivés (hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique et les polymères associatifs non ioniques, anioniques, cationiques ou amphotères, tels que les polymères commercialisés sous les appellations PEMULEN® TR1 ou TR2 par la société GOODRICH, SALCARE® SC90 par la société ALLIED COLLOIDS, ACULYN® 22, 28, 33, 44, ou 46 par la société ROHM & HAAS et ELFACOS® T210 et T212 par la société AKZO.

Ces épaississants d'appoint peuvent représenter de 0,01 à 10% en poids du poids total de la composition.

Un autre objet de l'invention est un procédé de teinture d'oxydation des fibres kératiniques humaines et en particulier des cheveux mettant en oeuvre une composition colorante comprenant dans un support approprié pour la teinture desdites fibres au moins un colorant d'oxydation et une composition oxydante telle que définie ci-dessus.

Selon ce procédé, on applique sur les fibres au moins une composition colorante telle que définie précédemment, la couleur étant révélée à pH acide, neutre ou alcalin à l'aide d'une composition oxydante selon l'invention qui est appliquée simultanément ou séquentiellement, avec ou sans rinçage intermédiaire.

Selon une forme de mise en oeuvre particulièrement préférée du procédé de teinture selon l'invention, on mélange, au moment de l'emploi, la composition colorante décrite ci-dessus avec une composition oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Un autre objet de la présente invention est un procédé de déformation permanente des fibres kératiniques humaines et en particulier des cheveux, utilisant comme composition oxydante la composition définie ci-dessus.

La première étape de ce procédé consiste à appliquer sur les cheveux une composition réductrice. Cette application se fait mèche par mèche ou globalement.

La composition réductrice comprend au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Les cheveux peuvent également être mis en forme sans l'aide de moyens extérieurs, simplement avec les doigts.

Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape, facultative, du procédé (étape (ii)), les cheveux imprégnés de la composition réductrice sont donc ensuite rincés soigneusement par une composition aqueuse.

Puis, dans une troisième étape (étape (iii)), on applique sur les cheveux ainsi rincés la composition oxydante selon la présente invention, dans le but de fixer la nouvelle forme imposée aux cheveux.

Comme dans le cas de l'application de la composition réductrice, la chevelure sur laquelle a été appliquée là composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées selon l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.

On obtient finalement une chevelure facile à démêler, douce. Les cheveux sont ondulés.

La composition oxydante selon l'invention peut également être utilisée dans un procédé de décoloration des fibres kératiniques humaines et en particulier des cheveux.

Le procédé de décoloration selon l'invention comprend une étape d'application sur les fibres kératiniques d'une composition oxydante selon l'invention, cette composition comprenant de préférence de l'eau oxygénée en milieu alcalin après mélange extemporané. Classiquement, une deuxième étape du procédé de décoloration selon l'invention est une étape de rinçage des fibres kératiniques.

Les exemples suivants illustrent l'invention sans présenter un caractère limitatif.

### EXEMPLE 1 :

On a préparé la composition de décoloration aqueuse prête à l'emploi suivante (quantités exprimées en grammes):

| | |
|---|---|
| Peroxyde d'hydrogène à 200 volumes | 12 |
| Stabilisant | qs |
| Polymère selon l'invention** | 0.3 MA* |
| Agent de pH qs | pH 4,7 |
| Eau qsp | 100 |

| | |
|---|---|
| MA*= Matière Active | |

Le polymère selon l'invention**1 est un terpolymère vinylpyrrolidone / diméthylaminopropylméthacrylamide/ chlorure de lauryldiméthylméthacrylamidoammonium proposé par la Société ISP sous la référence POLYMER ACP-1234.

La composition de décoloration ci-dessus a été appliquée et laissée 45 minutes sous casque sur des cheveux naturels, puis rincées abondamment à l'eau. On a obtenu un éclaircissement régulier de la chevelure.

### EXEMPLE 2 :

On a préparé la composition de décoloration suivante
( quantités exprimées en grammes):

| Composition anhydre | |
|---|---|
| Persulfate de potassium | 35 |
| Persulfate de sodium | 30 |
| Métasilicate de sodium | 14 |
| Chlorure d'ammonium | 5 |
| EDTA | 1 |
| Dioctylsulfosuccinate de sodium/benzoate de sodium | 1 |
| Stéarate de calcium | 1 |
| Polymère selon l'invention** | 3 MA* |
| Silice | 7 |

| | |
|---|---|
| MA*= Matière Active | |

Le polymère selon l'invention**1 est un terpolymère vinylpyrrolidone / diméthylaminopropylméthacrylamide/ chlorure de lauryldiméthylméthacrylamidoammonium proposé par la Société ISP sous la référence POLYMER ACP-1234.

40 g de la composition anhydre ci-dessus ont été mélangés avec 80 g de la composition aqueuse suivante :

| **Composition aqueuse** | |
|---|---|
| Alcool cétéarylique/ceteareth 30 | 2,85 |
| Stabilisants | 0,06 |
| Séquestrant | 0,15 |
| Peroxyde d'hydrogène à 200 volumes | 9 |
| Acide phosphorique qs | pH2 |
| Eau distillée qsp | 100 |

On a obtenu alors une crème décolorante prête à l'emploi qui, appliquée et laissée 45 minutes sous casque, a permis d'obtenir une décoloration puissante et homogène de cheveux naturels foncés.

### EXEMPLE 3:

Composition de déformation permanente:

On a préparé la composition réductrice suivante
(quantités exprimées en grammes) :

| | |
|---|---|
| Acide thioglycolique | 9,2 |
| Arginine | 15 |
| Ammoniaque à 20% d'NH3 | 9,3 |
| Carbonate d'ammonium | 4,5 |
| Cocoylamidopropylbétaïne/monolaurate de glycérol (25/5) en solution aqueuse à 30% | 1,3 |
| Peptisant | 0,8 |
| Alcool isostéarylique (TEGO ALKANOL 66 vendu par la société GOLDSCHMIDT) | 12 |
| Agent séquestrant | 0,4 |
| Parfum | 0,4 |
| Eau déminéralisée q.s.p. | 100 |

Cette composition réductrice a été appliquée sur une mèche de cheveux humides, préalablement enroulée sur un bigoudi de 9 mm de diamètre. Après 10 minutes de temps de pause, on l'a rincée abondamment à l'eau. On a ensuite appliqué la composition oxydante suivante :

### Composition oxydante:

- Terpolymère vinylpyrrolidone / diméthylaminopropylméthacrylamide/ chlorure de lauryldiméthylméthacrylamidoammonium proposé par la Société ISP sous la référence POLYMER ACP-1234 (Polymère selon l'invention) 1 g
- Eau oxygénée 8 volumes
- Pyrophosphate tétrasodique (0,02g) et stannate de sodium (0,04 g)
- Agent séquestrant : pentaacétate pentasodique 0,06 MA*
- Eau déminéralisée q.s.p. 100g
MA* désigne Matière Active

Après 10 minutes de temps de pause, on a rincé à nouveau abondamment la mèche. Puis on a déroulé les cheveux du bigoudi et on les a séchés. La mèche a été ondulée.

## Revendications

1. Composition cosmétique et/ou dermatologique destinée au traitement des matières kératiniques, **caractérisée par le fait qu'**elle comprend dans un support approprié pour les matières kératiniques :
(i) au moins un agent oxydant et,
(ii) un polymère poly(vinyllactame) cationique comprenant :
- a) au moins un monomère de type vinyl lactame ou alkylvinyllactame;
- b) au moins un monomère de structures (I) ou (II) suivantes : 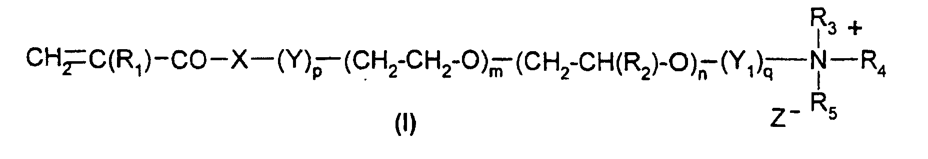 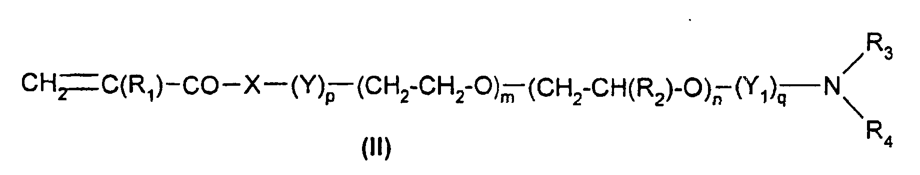
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (III) :
―(Y₂)ᵣ―(CH₂-CH(R₇)―O)ₓ―R₈ (III)
Y , Y₁ et Y₂ désignent, indépendamment l'un de l'autre; un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

2. Composition selon la revendication 1 **caractérisée par le fait que** le monomère vinyl lactame ou alkylvinyllactame est un composé de structure (IV) : 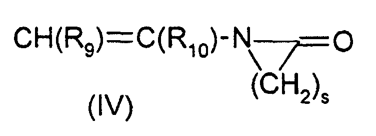 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

3. Composition selon la revendication 2 **caractérisée par le fait que** le monomère de formule (IV) est la vinylpyrrolidone.

4. Composition selon l'une quelconque des revendications précédentes, **caractérisée par le fait que** dans les formules (I) ou (II), les radicaux R₃, R₄, R₅ désignent, indépendamment l'un de l'autre, un atome d'hydrogène ou un radical alkyle linéaire ou ramifié en C₁-C₃₀.

5. Composition selon l'une quelconque des revendications précédentes, **caractérisée par le fait que** le monomère b) est un monomère de formule (I).

6. Composition selon la revendication 5, **caractérisée par le fait que** dans la formule (I), m et n sont égaux à zéro.

7. Composition selon l'une quelconque des revendications précédentes, **caractérisée par le fait que** le contre ion Z⁻ des monomères de formule (I) est choisi parmi les ions halogénures, les ions phosphates, l'ion méthosulfate, l'ion tosylate.

8. Composition selon l'une quelconque des revendications 1 à 7, **caractérisée par le fait que** le ou les polymères poly(vinyllactame) cationiques contiennent un ou plusieurs monomères supplémentaires cationiques ou non ioniques.

9. Composition selon la revendication 8, **caractérisée par le fait que** le poly(vinyllactame) cationique est un terpolymère comprenant :
a)-un monomère de formule (IV),
b)-un monomère de formule (I) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (II) dans laquelle R₃ et R₄ désignent, indépendamment l'un de l'autre, un atome d'hydrogène ou un radical alkyle en C₁-C₅.

10. Composition selon la revendication 9, **caractérisée par le fait que** le terpolymère comprend en poids, 40 à 95% de monomère (a), 0,25 à 50% de monomère (b) et 0,1 à 55% de monomère (c).

11. Composition selon l'une quelconque des revendications précédentes, **caractérisée par le fait que** les poly(vinyllactame) cationiques sont choisis parmi les terpolymères vinylpyrrolidone / diméthylaminopropylméthacrylamide / tosylate de dodécyldiméthylméthacrylamidopropylammonium, les terpolymères vinylpyrrolidone / diméthylaminopropylméthacrylamide / tosylate de cocoyldiméthylméthacrylamidopropylammonium, les terpolymères vinylpyrrolidone / diméthylaminopropylméthacrylamide / tosylate ou chlorure de lauryldiméthylméthacrylamidopropylammonium.

12. Composition selon l'une quelconque des revendications précédentes, **caractérisée par le fait que** la masse moléculaire en poids des poly(vinyllactame) cationiques est comprise entre 500 et 20 000 000, de préférence comprise entre 200 000 et 2 000 000 et plus préférentiellement comprise entre 400 000 et 800 000.

13. Composition selon l'une quelconque des revendications précédentes, **caractérisée par** le fait le ou les poly(vinyllactame) cationiques sont utilisés en une quantité variant de 0,01 à 30% en poids du poids total de la composition.

14. Composition selon la revendication 13, **caractérisée par** le fait le ou les poly(vinyllactame) cationiques sont utilisés en une quantité variant de 0,1 à 10% en poids du poids total de la composition.

15. Composition selon la revendication 14, **caractérisée par** le fait le ou les poly(vinyllactame) cationiques sont utilisés en une quantité variant de 0,5 à 2% en poids du poids total de la composition.

16. Composition selon l'une quelconque des revendications précédentes, **caractérisée par le fait que** l'agent oxydant est choisi dans le groupe formé par le peroxyde d'hydrogène, le peroxyde d'urée, les perborates, les persulfates, ou leurs mélanges.

17. Composition selon la revendication 16, **caractérisée par le fait que** l'agent oxydant est le peroxyde d'hydrogène.

18. Composition selon la revendication 17, **caractérisée par le fait que** la concentration en peroxyde d'hydrogène varie de 0,5 à 40 volumes et de préférence de 2 à 30 volumes.

19. Composition selon la revendication 18, **caractérisée par le fait que** l'agent oxydant est l'eau oxygénée.

20. Composition selon la revendication 19, **caractérisée par le fait que** l'eau oxygénée contient un agent stabilisant.

21. Composition selon la revendication 20, **caractérisée par le fait que** l'agent stabilisant est choisi parmi les pyrophosphates de métaux alcalins ou alcalino-terreux, les stannates des métaux alcalins ou alcalino-terreux, la phénacétine, ou les sels d'acides et d'oxyquinoléine.

22. Composition selon la revendication 21, **caractérisée par le fait que** l'agent stabilisant est un stannate ou un stannate associé à un pyrophosphate.

23. Composition selon l'une quelconque des revendications 20 à 22, **caractérisée par le fait que** la concentration en agent stabilisant varie de 0,0001 à 5% en poids et de préférence de 0,01 à 2% en poids par rapport au poids total de la composition.

24. Composition selon l'une quelconque des revendications 17 à 23, **caractérisée par le fait que** le rapport des concentrations du peroxyde d'hydrogène aux agents stabilisants varie de 0,05 à 1000, de préférence de 0,1 à 500 et plus particulièrement de 1 à 200.

25. Composition selon l'une quelconque des revendications 17 à 24, **caractérisée par le fait que** le rapport des concentrations du ou des poly(vinyllactame) cationiques définis selon les revendications 1 à 12, aux agents stabilisants, varie de 0,05 à 1000, de préférence de 0,1 à 500 et plus particulièrement de 1 à 200.

26. Composition selon l'une quelconque des revendications précédentes, **caractérisée par le fait que** le rapport des concentrations du ou des poly(vinyllactame) cationiques définis selon les revendications 1 à 12, aux agents oxydants, varie de 0,001 à 10, de préférence de 0,01 à 5 et plus particulièrement de 0,02 à 1.

27. Composition selon l'une quelconque des revendications précédentes, **caractérisée par le fait que** la concentration en oxydant varie de 0,1 à 25% en poids par rapport au poids total de la composition.

28. Composition selon l'une quelconque des revendications précédentes, **caractérisée par le fait qu'**elle est aqueuse et que le pH de l'ensemble de la composition varie de 1 à 13, et de préférence de 2 à 12.

29. Procédé de teinture d'oxydation des fibres kératiniques humaines et en particulier les cheveux mettant en oeuvre une composition colorante comprenant dans un support approprié pour la teinture des fibres kératiniques au moins un colorant d'oxydation et une composition oxydante telle que définie dans l'une quelconque des revendications 1 à 28.

30. Procédé de teinture selon la revendication 29 selon lequel on mélange, au moment de l'emploi, la composition colorante avec la composition oxydante ; le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

31. Procédé de teinture selon la revendication 30 selon lequel on applique séquentiellement la composition colorante et la composition oxydante, avec ou sans rinçage intermédiaire.

32. Procédé de traitement des fibres kératiniques humaines et en particulier les cheveux, en vue d'obtenir une déformation permanente de ces dernières, en particulier sous la forme de cheveux permanentés, ce procédé comprenant les étapes suivantes : (i) on applique sur la matière kératinique à traiter une composition réductrice, la fibre kératinique étant mise sous tension mécanique avant, pendant, ou après ladite application, (ii) on rince éventuellement la fibre kératinique, (iii) on applique sur la fibre kératinique éventuellement rincée une composition oxydante telle que définie à l'une des revendications 1 à 28, (iv) on rince éventuellement à nouveau la fibre kératinique.

33. Procédé de décoloration des fibres kératiniques humaines et en particulier les cheveux, comprenant les étapes suivantes : i) on applique sur la fibre kératinique une composition oxydante selon l'une quelconque des revendications 1 à 28, ii) on rince la fibre kératinique ainsi traitée.

34. Utilisation comme agent épaississant et/ou gélifiant dans une composition cosmétique et/ou dermatologique comprenant au moins un oxydant, d'un poly(vinyllactame) cationique tel que défini à l'une quelconque des revendications 1 à 12.

## Patentansprüche

1. Kosmetische und/oder dermatologische Zusammensetzung, die für die Behandlung von Keratinsubstanzen vorgesehen ist, **dadurch gekennzeichnet, dass** sie in einem für Keratinsubstanzen geeigneten Träger enthält:
(i) mindestens ein Oxidationsmittel und
(ii) ein kationisches Polyvinyllactam-Polymer, das enthält:
- a) mindestens ein Monomer vom Vinyllactamtyp oder Alkylvinyllactamtyp;
- b) mindestens ein Monomer der folgenden Strukturen (I) oder (II): 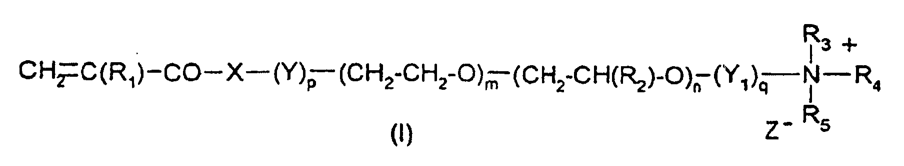 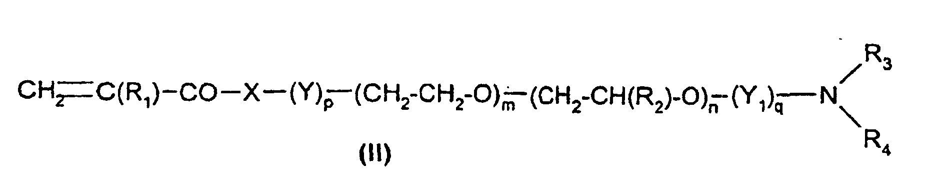
worin bedeuten:
X ein Sauerstoffatom oder eine Gruppe NR₆,
R₁ und R₆ unabhängig voneinander ein Wasserstoffatom oder eine geradkettige oder verzweigte C₁₋₅-Alkylgruppe,
R₂ eine geradkettige oder verzweigte C₁₋₄-Alkylgruppe,
R₃, R₄ und R₅ unabhängig voneinander ein Wasserstoffatom, eine geradkettige oder verzweigte C₁₋₃₀-Alkylgruppe oder eine Gruppe der Formel (III):
―(Y₂)ᵣ―(CH₂-CH(R₇)-O)ₓ―R₈
worin Y, Y₁ und Y₂ unabhängig voneinander eine geradkettige oder verzweigte C₂₋₁₆-Alkylengruppe bedeuten,
R₇ ein Wasserstoffatom, eine geradkettige oder verzweigte C₁₋₄-Alkylgruppe oder eine geradkettige oder verzweigte C₁₋₄-Hydroxyalkylgruppe,
R₈ ein Wasserstoffatom oder eine geradkettige oder verzweigte C₁₋₃₀-Alkylgruppe,
p, q und r unabhängig voneinander entweder Null oder 1,
m und n unabhängig voneinander Null oder eine ganze Zahl von 1 bis 100,
x eine ganze Zahl von 1 bis 100,
Z ein Anion einer organischen oder anorganischen Säure,
mit der Maßgabe, dass:
- mindestens einer der Substituenten R₃, R₄, R₅ oder R₈ eine geradkettige oder verzweigte C₉₋₃₀-Alkylgruppe bedeutet,
- wenn m oder n von Null verschieden ist, q 1 bedeutet,
- wenn m oder n Null sind, p oder q 0 bedeutet.

2. Zusammensetzung nach Anspruch 1, **dadurch gekennzeichnet, dass** das Vinyllactam- oder Alkylvinyllactam-Monomer eine Verbindung der folgenden Struktur (IV) ist: 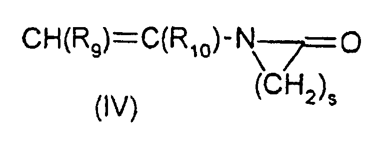 worin bedeuten:
s eine ganze Zahl von 3 bis 6,
R₉ eine Wasserstoffatom oder eine C₁₋₅-Alkylgruppe,
R₁₀ eine Wasserstoffatom oder eine C₁₋₅-Alkylgruppe,
mit der Maßgabe, dass mindestens eine Gruppe R₉ oder R₁₀ ein Wasserstoffatom bedeutet.

3. Zusammensetzung nach Anspruch 2, **dadurch gekennzeichnet, dass** das Monomer der Formel (IV) das Vinylpyrrolidon ist.

4. Zusammensetzung nach einem der vorhergehenden Ansprüche, **dadurch gekennzeichnet, dass** in den Formeln (I) oder (II) die Gruppen R₃, R₄ und R₅ unabhängig voneinander ein Wasserstoffatom oder eine geradkettige oder verzweigte C₁₋₃₀-Alkylgruppe bedeuten.

5. Zusammensetzung nach einem der vorhergehenden Ansprüche, **dadurch gekennzeichnet, dass** das Monomer b) ein Monomer der Formel (I) ist.

6. Zusammensetzung nach Anspruch 5, **dadurch gekennzeichnet, dass** in der Formel (I) m und n Null bedeuten.

7. Zusammensetzung nach einem der vorhergehenden Ansprüche, **dadurch gekennzeichnet, dass** das Gegenion Z⁻ der Monomere der Formel (I) unter den Halogeniden, Phosphaten, Methosulfat und Tosylat ausgewählt ist.

8. Zusammensetzung nach einem der Ansprüche 1 bis 7, **dadurch gekennzeichnet, dass** das oder die kationische(n) Polyvinyllactam-Polymer(e) ein oder mehrere zusätzliche kationische oder nichtionische Monomere enthalten.

9. Zusammensetzung nach Anspruch 8, **dadurch gekennzeichnet, dass** das kationische Polyvinyllactam ein Terpolymer ist, das umfasst:
a) ein Monomer der Formel (IV),
b) ein Monomer der Formel (I) mit p=1 und q=0, wobei R₃ und R₄ unabhängig voneinander ein Wasserstoffatom oder eine C₁₋₅-Alkylgruppe bedeuten und R₅ eine C₉₋₂₄-Alkylgruppe ist, und
c) ein Monomer der Formel (II), wobei R₃ und R₄ unabhängig voneinander ein Wasserstoffatom oder eine C₁₋₅-Alkylgruppe bedeuten.

10. Zusammensetzung nach Anspruch 9, **dadurch gekennzeichnet, dass** das Terpolymer 40 bis 95 Gew.-% Monomer (a), 0,25 bis 50 Gew.-% Monomer (b) und 0,1 bis 55 Gew.-% Monomer (c) enthält.

11. Zusammensetzung nach einem der vorhergehenden Ansprüche, **dadurch gekennzeichnet, dass** die kationischen Polyvinyllactame unter den Terpolymeren Vinylpyrrolidon/Dimethylaminopropylmethacrylamid/Dodecyldimethylmethacrylamidopropylammoniumtosylat, den Terpolymeren Vinylpyrrolidon/Dimethylaminopropylmethacrylamid/Cocoyldimethylmethacrylamidopropylammoniumtosylat und den Terpolymeren Vinylpyrrolidon/Dimethylaminopropylmethacrylamid/Lauryldimethylmethacrylamidopropylammonioumtosylat oder Lauryldimethylmethacrylamidopropylammoniumchlorid ausgewählt sind.

12. Zusammensetzung nach einem der vorhergehenden Ansprüche, **dadurch gekennzeichnet, dass** das Molekulargewicht der kationischen Polyvinyllactame im Bereich von 500 bis 20 000 000, vorzugsweise 200 000 bis 2 000 000 und besonders bevorzugt 400 000 bis 800 000 liegt.

13. Zusammensetzung nach einem der vorhergehenden Ansprüche, **dadurch gekennzeichnet, dass** das oder die kationische(n) Polyvinyllactam(e) in einer Menge von 0,01 bis 30 Gew.-% des Gesamtgewichts der Zusammensetzung verwendet werden.

14. Zusammensetzung nach Anspruch 13, **dadurch gekennzeichnet, dass** das oder die kationische(n) Polyvinyllactam(e) in einer Menge von 0,1 bis 10 Gew.-% des Gesamtgewichts der Zusammensetzung verwendet werden.

15. Zusammensetzung nach Anspruch 14, **dadurch gekennzeichnet, dass** das oder die kationische(n) Polyvinyllactam(e) in einer Menge von 0,5 bis 2 Gew.- des Gesamtgewichts der Zusammensetzung verwendet werden.

16. Zusammensetzung nach einem der vorhergehenden Ansprüche, **dadurch gekennzeichnet, dass** das Oxidationsmittel unter Wasserstoffperoxid, Harnstoffperoxid, Perboraten, Persulfaten oder deren Gemischen ausgewählt ist.

17. Zusammensetzung nach Anspruch 16, **dadurch gekennzeichnet, dass** das Oxidationsmittel Wasserstoffperoxid ist.

18. Zusammensetzung nach Anspruch 17, **dadurch gekennzeichnet, dass** die Wasserstoffperoxidkonzentration im Bereich von 0,5 bis 40 Volumina und vorzugsweise 2 bis 30 Volumina liegt.

19. Zusammensetzung nach Anspruch 18, **dadurch gekennzeichnet, dass** das Oxidationsmittel eine wässrige Wasserstoffperoxidlösung ist.

20. Zusammensetzung nach Anspruch 19, **dadurch gekennzeichnet, dass** die Wasserstoffperoxidlösung einen Stabilisator enthält.

21. Zusammensetzung nach Anspruch 20, **dadurch gekennzeichnet, dass** der Stabilisator unter den Alkali- oder Erdalkalipyrophosphaten, Alkali- oder Erdalkalistannaten, Phenacetin oder den Salzen von Säuren und Oxychinolin ausgewählt ist.

22. Zusammensetzung nach Anspruch 21, **dadurch gekennzeichnet, dass** der Stabilisator ein Stannat oder ein Stannat in Kombination mit einem Pyrophosphat ist.

23. Zusammensetzung nach einem der Ansprüche 20 bis 22, **dadurch gekennzeichnet, dass** die Konzentration des Stabilisators im Bereich von 0,0001 bis 5 Gew.-% und vorzugsweise 0,01 bis 2 Gew.-%, bezogen auf das Gesamtgewicht der Zusammensetzung, liegt.

24. Zusammensetzung nach einem der Ansprüche 17 bis 23, **dadurch gekennzeichnet, dass** das Verhältnis der Konzentration von Wasserstoffperoxid und der Stabilisatoren im Bereich von 0,05 bis 1 000, vorzugsweise 0,1 bis 500 und insbesondere 1 bis 200 liegt.

25. Zusammensetzung nach einem der Ansprüche 17 bis 24, **dadurch gekennzeichnet, dass** das Verhältnis der Konzentration des oder der kationischen Polyvinyllactame gemäß den Ansprüchen 1 bis 12 und der Stabilisatoren im Bereich von 0,05 bis 1 000, vorzugsweise 0,1 bis 500 und insbesondere 1 bis 200 liegt.

26. Zusammensetzung nach einem der vorhergehenden Ansprüche, **dadurch gekennzeichnet, dass** das Verhältnis der Konzentration des oder der kationischen Polyvinyllactame gemäß den Ansprüchen 1 bis 12 und der Oxidationsmittel im Bereich von 0,001 bis 10, vorzugsweise 0,01 bis 5 und insbesondere 0,02 bis 1 liegt.

27. Zusammensetzung nach einem der vorhergehenden Ansprüche, **dadurch gekennzeichnet, dass** die Konzentration des Oxidationsmittels im Bereich von 0,1 bis 25 Gew.-%, bezogen auf das Gesamtgewicht der Zusammensetzung, liegt.

28. Zusammensetzung nach einem der vorhergehenden Ansprüche, **dadurch gekennzeichnet, dass** sie wässrig ist und der pH-Wert der gesamten Zusammensetzung im Bereich von 1 bis 13 und vorzugsweise 2 bis 12 liegt.

29. Verfahren zum oxidativen Färben von menschlichen Keratinfasern und insbesondere Haaren unter Verwendung einer färbenden Zusammensetzung, die in einem zum Färben von Keratinfasem geeigneten Träger mindestens einen Oxidationsfarbstoff und eine oxidierende Zusammensetzung nach einem der Ansprüche 1 bis 28 enthält.

30. Verfahren zum Färben nach Anspruch 29, wobei bei der Anwendung die färbende Zusammensetzung mit der oxidierenden Zusammensetzung vermischt wird; das erhaltene Gemisch anschließend auf die Keratinfasern aufgetragen und etwa 3 bis 50 min und vorzugsweise 5 bis 30 min einwirken gelassen wird, worauf gespült, mit Haarwaschmittel gewaschen, nochmals gespült und getrocknet wird.

31. Verfahren zum Färben nach Anspruch 30, wobei die färbende Zusammensetzung und die oxidierende Zusammensetzung mit oder ohne zwischenzeitlichen Spülschritt nacheinander aufgetragen werden.

32. Verfahren zur Behandlung von menschlichen Keratinfasern und insbesondere Haaren für die dauerhafte Verformung der Keratinfasem, insbesondere in Form von dauergewellten Haaren, wobei das Verfahren die folgenden Schritte umfasst: (i) auf die zu behandelnden Keratinsubstanzen wird eine reduzierende Zusammensetzung aufgetragen, wobei die Keratinfasern vor, während oder nach dem Auftragen mechanisch unter Spannung gesetzt werden, (ii) gegebenenfalls werden die Keratinfasern gespült, (iii) auf die gegebenenfalls gespülten Keratinfasern wird eine oxidierende Zusammensetzung nach einem der Ansprüche 1 bis 28 aufgetragen, und (iv) gegebenenfalls werden die Keratinfasern nochmals gespült.

33. Verfahren zum Bleichen von menschlichen Keratinfasern und insbesondere Haaren, das die folgenden Schritte umfasst: (i) auf die Keratinfasern wird eine oxidierende Zusammensetzung nach einem der Ansprüche 1 bis 28 aufgetragen und (ii) die auf diese Weise behandelten Keratinfasern werden gespült.

34. Verwendung eines kationischen Polyvinyllactams nach einem der Ansprüche 1 bis 12 als Verdickungsmittel und/oder Gelbildner in einer kosmetischen und/oder dermatologischen Zusammensetzung, die mindestens ein Oxidationsmittel enthält.

## Claims

1. Cosmetic and/or dermatological composition intended for the treatment of keratin materials, **characterized in that** it comprises in an appropriate carrier for keratin materials:
(i) at least one oxidizing agent, and
(ii) a cationic poly(vinyllactam) polymer comprising:
-a) at least one monomer of the vinyllactam or alkylvinyllactam type;
-b) at least one monomer having the following structures (I) or (II): 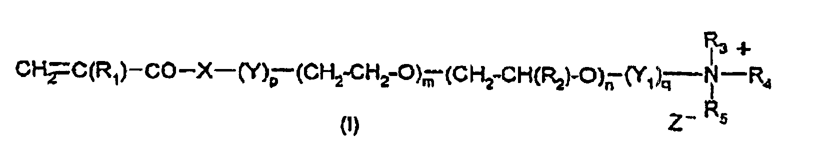 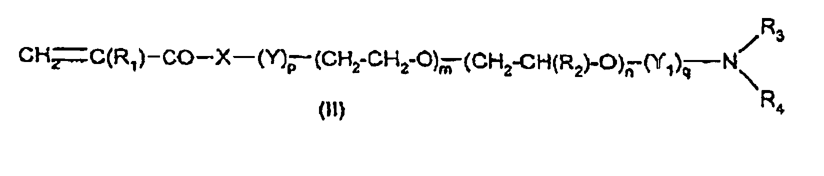
in which :
X denotes an oxygen atom or a radical NR₆,
R₁ and R₆ denote, independently of each other, a hydrogen atom or a linear or branched C₁-C₅ alkyl radical,
R₂ denotes a linear or branched C₁-C₄ alkyl radical,
R₃, R₄ and R₅ denote, independently of each other, a hydrogen atom, a linear or branched C₁-C₃₀ alkyl radical or a radical of formula (III):
―(Y₂)ᵣ―(CH₂-CH(R₇)-O)ₓR₈ (III)
Y, Y₁ and Y₂ denote, independently of each other, a linear or branched C₂-C₁₆ alkylene radical,
R₇ denotes a hydrogen atom, or a linear or branched C₁-C₄ alkyl radical or a linear or branched C₁-C₄ hydroxyalkyl radical,
R₉ denotes a hydrogen atom or a linear or branched C₁-C₃₀ alkyl radical,
p, q and r denote, independently of each other, either the value zero, or the value 1,
m and n denote, independently of each other, an integer ranging from 0 to 100,
x denotes an integer ranging from 1 to 100,
Z denotes an organic or inorganic acid anion,
provided that:
- at least one of the substituents R₃, R₄, R₅ or R₈ denotes a linear or branched C₉-C₃₀ alkyl radical,
- if m or n is different from zero, then q is equal to 1,
- if m or n are equal to zero, then p or q is equal to 0.

2. Composition according to Claim 1, **characterized in that** the vinyllactam or alkylvinyllactam monomer is a compound having the structure (IV): 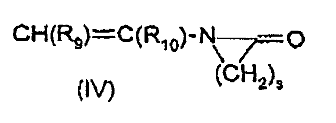 in which:
s denotes an integer ranging from 3 to 6,
R₉ denotes a hydrogen atom or a C₁-C₅ alkyl radical,
R₁₀ denotes a hydrogen atom or a C₁-C₅ alkyl radical,
provided that at least one of the radicals R₉ and R₁₀ denotes a hydrogen atom.

3. Composition according to Claim 2, **characterized in that** the monomer of formula (IV) is vinylpyrrolidone.

4. Composition according to any one of the preceding claims, **characterized in that** in formulae (I) or (II), the radicals R₃, R₄ and R₅ denote, independently of each other, a hydrogen atom or a linear or branched C₁-C₃₀ alkyl radical.

5. Composition according to any one of the preceding claims, **characterized in that** the monomer b) is a monomer of formula (I).

6. Composition according to Claim 5, **characterized in that** in formula (I), m and n are equal to zero.

7. Composition according to any one of the preceding claims, **characterized in that** the counterion Z⁻ of the monomers of formula (I) is chosen from the halide ions, the phosphate ions, the methosulphate ion and the tosylate ion.

8. Composition according to any one of Claims 1 to 7, **characterized in that** the cationic poly-(vinyllactam) polymer(s) contain one or more additional cationic or nonionic monomers.

9. Composition according to Claim 8, **characterized in that** the cationic poly(vinyllactam) is a terpolymer comprising:
a) - one monomer of formula (IV),
b) - one monomer of formula (I) in which p = 1, q = 0, R₃ and R₄ denote, independently of each other, a hydrogen atom or a C₁-C₅ alkyl radical and R₅ denotes a C₉-C₂₄ alkyl radical, and
c) - one monomer of formula (II) in which R₃ and R₄ denote, independently of each other, a hydrogen atom or a C₁-C₅ alkyl radical.

10. Composition according to Claim 9, **characterized in that** the terpolymer comprises, by weight, 40 to 95% of monomer (a), 0.25 to 50% of monomer (b) and 0.1 to 55% of monomer (c).

11. Composition according to any one of the preceding claims, **characterized in that** the cationic poly(vinyllactams) are chosen from the terpolymers vinylpyrrolidone/dimethylaminopropylmethacrylamide/dodecyldimethylmethacrylamidopropylammonium tosylate, the terpolymers vinylpyrrolidone/dimethylaminopropylmethacrylamide/cocoyldimethylmethacrylamidopropylammonium tosylate, the terpolymers vinylpyrrolidone/dimethylaminopropylmethacrylamide/lauryldimethylmethacrylamidopropylammonium tosylate or chloride.

12. Composition according to any one of the preceding claims, **characterized in that** the weight-average molecular mass of the cationic poly(vinyllactams) is between 500 and 20 000 000, preferably between 200 000 and 2 000 000 and more preferably between 400 000 and 800 000.

13. Composition according to any one of the preceding claims, **characterized in that** the cationic poly(vinyllactam) or poly(vinyllactams) are used in a quantity varying from 0.01 to 30% by weight of the total weight of the composition.

14. Composition according to Claim 13, **characterized in that** the cationic poly(vinyllactam) or poly (vinyllactams) are used in a quantity varying from 0.1 to 10% by weight of the total weight of the composition.

15. Composition according to Claim 14, **characterized in that** the cationic poly(vinyllactam) or poly(vinyllactams) are used in a quantity varying from 0.5 to 2% by weight of the total weight of the composition.

16. Composition according to any one of the preceding claims, **characterized in that** the oxidizing agent is chosen from the group consisting of hydrogen peroxide, urea peroxide, perborates, persulphates or mixtures thereof.

17. Composition according to Claim 16, **characterized in that** the oxidizing agent is hydrogen peroxide.

18. Composition according to Claim 17, **characterized in that** the hydrogen peroxide concentration varies from 0.5 to 40 volumes, and preferably from 2 to 30 volumes.

19. Composition according to Claim 18, **characterized in that** the oxidizing agent is hydrogen peroxide.

20. Composition according to Claim 19, **characterized in that** the hydrogen peroxide contains a stabilizing agent.

21. Composition according to Claim 20, **characterized in that** the stabilizing agent is chosen from alkali or alkaline-earth metal pyrophosphates, alkali or alkaline-earth metal stannates, phenacetin, or the acid and oxyquinoline salts.

22. Composition according to Claim 21, **characterized in that** the stabilizing agent is a stannate or a stannate combined with a pyrophosphate.

23. Composition according to any one of Claims 20 to 22, **characterized in that** the stabilizing agent concentration varies from 0.0001 to 5% by weight and preferably from 0.01 to 2% by weight relative to the total weight of the composition.

24. Composition according to any one of Claims 17 to 23, **characterized in that** the ratio of the concentrations of hydrogen peroxide to the stabilizing agents varies from 0.05 to 1000, preferably from 0.1 to 500 and more particularly from 1 to 200.

25. Composition according to any one of Claims 17 to 24, **characterized in that** the ratio of the concentrations of the cationic poly(vinyllactam) or poly(vinyllactams) defined according to Claims 1 to 12 to the stabilizing agents varies from 0.05 to 1000, preferably from 0.1 to 500 and more particularly from 1 to 200.

26. Composition according to any one of the preceding claims, **characterized in that** the ratio of the concentrations of the cationic poly(vinyllactam) or poly(vinyllactams) defined according to Claims 1 to 12 to the oxidizing agents varies from 0.001 to 10, preferably from 0.01 to 5 and more particularly from 0.02 to 1.

27. Composition according to any one of the preceding claims, **characterized in that** the oxidizing agent concentration varies from 0.1 to 25% by weight relative to the total weight of the composition.

28. Composition according to any one of the preceding claims, **characterized in that** it is aqueous and **in that** the pH of the whole composition varies from 1 to 13, and preferably from 2 to 12.

29. Method for the oxidation dyeing of human keratin fibres, and in particular the hair, using a dyeing composition comprising; in an appropriate carrier for dyeing keratin fibres, at least one oxidation dye and an oxidizing composition as defined in any one of Claims 1 to 28.

30. Dyeing method according to Claim 29, according to which the dyeing composition is mixed, at the time of use, with the oxidizing composition; the mixture obtained is then applied to keratin fibres and left in for 3 to 50 minutes approximately, preferably for 5 to 30 minutes approximately, after which they are rinsed, washed with shampoo, rinsed again and dried.

31. Dyeing method according to Claim 30, according to which the dyeing composition and the oxidizing composition are applied successively, with or without intermediate rinsing.

32. Method for treating human keratin fibres, and in particular the hair, in order to obtain a permanent reshaping thereof, in particular in the form of permanently waved hair, this method comprising the following steps: (i) a reducing composition is applied to the keratin material to be treated, the keratin fibre being subjected to mechanical stress before, during or after the said application, (ii) the keratin fibre is optionally rinsed, (iii) an oxidizing composition as defined in one of Claims 1 to 28 is applied to the optionally rinsed keratin fibre, (iv) the keratin fibre is optionally rinsed again.

33. Method for bleaching human keratin fibres, and in particular the hair, comprising the following steps: (i) an oxidizing composition according to any one of Claims 1 to 28 is applied to the keratin fibre, (ii) the keratin fibre thus treated is rinsed.

34. Use of a cationic poly(vinyllactam) as defined in any one of Claims 1 to 12 as thickening and/or gelling agent in a cosmetic and/or dermatological composition comprising at least one oxidizing agent.
